# EUROPÄISCHE PATENTSCHRIFT

(11) **EP 4 313 229 B1**
(45) Veröffentlichungstag und Bekanntmachung des Hinweises auf die Patenterteilung: **20.08.2025**
(21) Anmeldenummer: 22717579.1
(22) Anmeldetag: 23.03.2022
(51) Int. Cl.: A61M 16/04, A61M 16/10

(54) **ANORDNUNG IN ART EINES SPRECHVENTILS ZUM AUFSETZEN UND ANBRINGEN AN EINE TRACHEOSTOMIEKANÜLE**
ARRANGEMENT IN THE MANNER OF A VOICE VALVE FOR PLACEMENT AND ATTACHMENT TO A TRACHEOSTOMY CANNULA
AGENCEMENT DU TYPE D'UNE VANNE DE PHONATION DESTINÉ À LA MISE EN PLACE ET À LA FIXATION À UNE CANULE DE TRACHÉOTOMIE

(30) Priorität: 24.03.2021 EP 21164568
(43) Veröffentlichungstag der Anmeldung: 07.02.2024
(73) Patentinhaber: Albert-Ludwigs-Universität Freiburg, 79098 Freiburg (DE)
(72) Erfinder: STAHL, Claudius, 79194 Heuweiler (DE); LÜCKING, Klaus-Michael, 79100 Freiburg (DE); TAUBER, Falk, 79110 Freiburg (DE); SPECK, Thomas, 79227 Schallstadt (DE); PHILIPP, Auth, 79115 Freiburg (DE); KNORR, Noah, 79104 Freiburg (DE)
(74) Vertreter: Rösler, Uwe
(86) Internationale Anmeldenummer: PCT/EP2022/057574
(87) Internationale Veröffentlichungsnummer: WO 2022/200401

(56) Entgegenhaltungen:
- EP-A1- 0 078 685
- US-A- 5 765 560
- US-A1- 2014 305 440

## Beschreibung

### Technisches Gebiet

Die Erfindung bezieht sich auf eine Sprechventil-Anordnung zum Aufsetzen und Anbringen an eine Tracheostomiekanüle, mit einem einen Durchströmungskanal umschließenden Durchströmungskörper, der eine Durchströmungskörperwand aufweist, die den Durchströmungskanal radial einschließt, und der einseitig endseitig eine geeignet ausgebildete Verbindungsstruktur zur Fügung an ein proximales Ende der Tracheostomiekanüle aufweist, an dessen der Verbindungsstruktur längs des Durchströmungskanals gegenüberliegenden Ende ein Einwegventil angeordnet ist und der zwischen der Verbindungsstruktur und dem Einwegventil eine durch eine Überdruckventilanordnung abschließbare Ventilöffnung vorsieht.

Tracheostomiekanülen dienen dem direkten Zugang zur Luftröhre einer Person unter Umgehung des Mund-, Rachen- sowie Kehlkopfbereiches zum Zwecke einer direkten Luftzu- und -abführung in bzw. aus der Lunge. Neben sogenannten ungeblockten Tracheostomiekanülen, die vorwiegend bei spontan atmenden Patienten mit konstantem, reflektorischen und aspirationsfreiem Schlucken eingesetzt werden, dienen sogenannte geblockte Tracheostomiekanülen, die distalseitig einen dilatierfähigen Cuff vorsehen, zur invasiven Beatmung und/oder der Beatmung aspirationsgefährdeter Patienten, bei denen sicherzustellen ist, dass ein unkontrolliertes Entweichen von Atemgasen unter Beatmung durch den Rachen und Mundraum vermieden wird und zudem zu verhindern ist, dass insbesondere bei Patienten mit Schluckstörungen Speisereste oder Speichel ungehindert in die unteren Luftwege oder in die Lunge geraten.

Das offen ausgebildete, proximale Ende einer Tracheostomiekanüle verfügt über einen geeignet ausgebildeten Anschlussflansch zum Anbringen beispielsweise eines Beatmungsgerätes, einer Tubusverlängerung oder für den Aufsatz eines die Einatemluft wärmenden und befeuchtenden Durchströmungskörpers, kurz HME (Heat and Moisture-Exchanger) oder eines sogenannten Sprechventils.

Ein Sprechventil ermöglicht das Sprechen, sofern die Stimmbänder sowie die oberen Atemwege intakt und funktionsfähig sind. Ein Sprechventil ist ein Einwegventil, das so aufgebaut ist, dass es sich beim Einatmen öffnet und den Luftstrom über die Tracheostomiekanüle in die Lunge leitet und beim Ausatmen hingegen verschließt, so dass die Ausatemluft durch den Kehlkopf über die Stimmbänder zu deren Schallanregung geleitet wird. Hierzu ist es jedoch erforderlich, dass die Tracheostomiekanüle entblockt wird, d.h. der am distalen Ende der Tracheostomiekanüle angeordnete Cuff ist zu entleeren, so dass sich ein ausreichend großer Zwischenspalt zwischen Cuff und Luftröhre auszubilden vermag.

Alternativ oder in Kombination zur Maßnahme der ungeblockten Tracheostomiekanüle unterstützen sogenannte gesiebte oder gefensterte Tracheostomiekanülen während der Ausatmung ein Überströmen der Stimmbänder mit Ausatemluft bei Verwendung eines Sprechventils.

Ein Problem entsteht jedoch bei der Verwendung eines Sprechventils mit einer nichtgefensterten Tracheostomiekanüle, wenn die Cuff versehentlich nicht entleert ist, d.h. die Tracheostomiekanüle befindet sich im geblockten Zustand, so dass es keinen Weg für die Ausatemluft des Patienten gibt. In diesem Fall erlaubt das Sprechventil zwar eine Einatmung, aber keine Ausatmung. Innerhalb weniger Atemzüge ist die Lunge maximal überbläht. Der Patient droht einerseits zu ersticken, da er nicht mehr atmen kann, und andererseits besteht die akute Gefahr eines Platzens der Lunge (Pneumothorax), etwa durch exzessiven Überdruck beim Husten.

Zu einer ähnlichen Situation kann es gelegentlich auch bei ungeblockter Tracheostomiekanüle kommen, wenn die Schleimhaut um die Tracheostomiekanüle unerwartet anschwillt und sich dadurch das Lumen zwischen Luftröhre und Tracheostomiekanüle verengt.

Zur Begegnung dieser Gefahren, die insbesondere ein Patient beim unmittelbaren Aufsetzen eines Sprechventils an einer geblockten und weder gefensterten noch gesiebten Tracheostomiekanüle ausgesetzt ist, wird in der Druckschrift WO 2016/139441 A1 vorgeschlagen, im Bereich des Sprechventilaufsatzes zwischen der Verbindung zur Tracheostomiekanüle und dem Einwegventil des Sprechventilsaufsatzes ein Überdruckventil anzuordnen, dessen Ventilfunktion derart eingestellt ist, dass das Überdruckventil bei für den Patienten ungefährlichen Druckverhältnissen innerhalb der Sprechventilanordnung fluid- bzw. gasdicht abschließt und ausschließlich bei Überschreiten eines vorgegebenen Überdruckes innerhalb der Sprechventilanordnung spontan auszulösen vermag, wodurch eine Ventilöffnung freigegeben wird, über die überschüssige Ausatemluftanteile in die Umgebung weitgehen widerstandsfrei abströmen kann.

Das bekannte Überdruckventil ist in Form eines Kugelventils ausgebildet, das aufgrund der Eigenschwere der Kugel eine Ventilöffnung gasdicht abzuschließen vermag. Größe, Gewicht und Anordnung der Kugel sind dabei derart bemessen, so dass im Falle einer für den Patienten gefährlichen Überdrucksituation die Kugel entgegen der Schwerkraft angehoben wird, wodurch die Entlüftungsöffnung freigegeben wird.

Auch das in der Druckschrift EP 2 908 895 B1 beschriebene Sprechventil für Tracheostomiekanülen verfügt neben der für Sprechventile charakteristischen Einwegventilfunktion über die Möglichkeit einer Entlüftung durch manuelles Überführen eines Öffnungsmechanismus in eine geöffnete Position, in der ein freies Atmen ermöglicht wird, wie es bei einer Tracheostomiekanüle ohne aufgebrachtes Sprechventil der Fall ist. Zum Öffnen ist ein aktives, zielgerichtetes Handeln des Patienten erforderlich, was bei intensivmedizinisch behandelten Patienten nicht zwingend vorausgesetzt werden kann.

Der Druckschrift EP 2 326 376 B1 ist eine kombinierte Ventilanordnung zur Anbringung an eine Tracheostomiekanüle zu entnehmen, die zu Zwecken der künstlichen bzw. mechanischen Beatmung über ein Inspirationsventil sowie über ein getrennt dazu ausgebildetes Expirationsventil verfügt, wobei letzteres als einstellbares PEEP-Ventil (positiver Endex spiratorischer Druck) ausgebildet ist. Insbesondere handelt es sich bei dem Expirations-PEEP-Ventil um ein Kugel-Käfig-Ventil mit Feder, über deren Spannung der Luftstromwiderstand des Expirations-PEEP-Ventils einstellbar ist.

Die Druckschrift EP 0 617 630 B1 offenbart ein Tracheostomventil, das über ein kombiniertes Inspirations- und Expirationsventil verfügt, die beide in Form einseitig, jeweils um eine Achse bidirektional schwenkbar gelagerte, flächige Schließglieder ausgebildet sind. Beide Schließglieder vermögen in gegenseitiger Überlappung das Tracheostomaventil vollständig zu schließen und je nach Luftdruckverhältnisse innerhalb des Ventils den Luftströmungsweg teilweise oder vollständig zu öffnen.

Die Druckschrift EP 0078685 A1 beschreibt eine Anordnung mit einem Ventil zur Steuerung der Luftströmung durch das Tracheostoma in Abhängigkeit vom Atemluftströmungsdruck sowie eine Einrichtung zur lösbaren Befestigung des Ventils mit dem Stoma. Das Ventil weist eine flexible Membran auf, die zwischen einer ersten, einer zweiten und einer dritten Stellung beweglich ist, wobei die erste Stellung für normale Atmung durch das Ventil benutzt wird, die flexible Membran durch stimmhafte Ausatmung in die zweite Stellung bewegt wird, in der sie am Ventilsitz angreift, wodurch der Luftströmung durch das Ventil unterbrochen wird, und die flexible Membran durch Husten in der Luftröhre erzeugten Druck für eine Luftströmung durch das Ventil hindurch in die dritte Stellung bewegt wird.

Die Druckschrift US 2014/0305440 A1 offenbart ein Beatmungsventil, das mit einer Tracheostomiekanüle verbunden ist und einen rohrförmigen Ventilkörpers aufweist, an dessen proximalen Ende ein mit Öffnungen versehenes Scheibenendstück mit einem O-Ring am Umfang lösbar eingesetzt ist. Eine schlaffe Membran, die über der Innenseite des Scheibenendstücks liegt, dient als Einlassventil, um die Inhalation und Luftaufnahme des Patienten zu ermöglichen, und als Rückschlagventil, um die Ausatmung des Patienten zu blockieren und sie so zum Kehlkopf, zu den Nebenhöhlen und zum Mund des Patienten zu leiten, damit dieser normal sprechen kann. Der O-Ring gibt bei kraftvoller Ausatmung des Patienten nach, um das Scheibenendstück aus dem röhrenförmigen Ventilkörper zu lösen. Das Beatmungsventil selbst ist ebenfalls an der Trachealkanüle befestigt, um zu verhindern, dass es nach einer Trennung der Verbindung verrutscht. Das Einatmungsventil weist zudem eine Pfeife auf, die ein hörbares Signal erzeugt, wenn die Ausatmung kräftig ist.

Die Druckschrift US 5,765,560 A offenbart ein Tracheostomventil mit einem einen Durchströmungskanal umschließenden Durchströmungskörper, dessen ein Ende mit einer Trachealkanüle verbindbar und an dessen anderen Ende eine Kippventilanordnung angebracht ist, die sich aus zwei jeweils um eine Drehachse schwenkbar gelagerte, sich in der Flächengröße unterscheidende Flächenelemente zusammensetzt. Das in der Fläche kleiner ausgestaltete Flächenelement dient als Einwegventil und stellt im geöffneten Zustand die Inspiration, die durch eine im größeren Flächenelement eingebrachte Atemöffnung erfolgt, sicher und ermöglicht im geschlossenen Zustand das Überströmen des Atemluftstromes über die Stimmbänder, wobei das kleinere Flächenelement die im größeren Flächenelement eingebrachte Atmenöffnung abdeckt. Im Falle eines auftretenden Überdruckes schwenken beide Flächenelemente im Verbund gemeinsam in eine das Tracheostomventil maximal öffnende Position.

Trotz der auf dem Markt verfügbaren Sprechventilen sowie der Kenntnis und den informellen Hinweisen, Sprechventilaufsätze nur auf ungeblockte Trachealkanülen aufzusetzen, kommt es weltweit nach wie vor zu Todesfällen bedingt durch das Aufsetzen eines Sprechventilaufsatzes auf eine geblockte Tracheostomiekanüle, und zwar auch dann, wenn erfahrene Operateure bzw. Betreuer die Kanüle aufsetzen. Das Bundesinstitut für Arzneimittel und Medizinprodukte hat infolgedessen im Juli 2019 auf diesen Gebrauchstauglichkeitsmangel verwiesen und zwingend eindeutige Warnhinweise auf Sprechventilaufsätzen verlangt. Selbst der Einsatz von als Kugelventile ausgebildeten Überdruckventilen im Bereich der Sprechventilanordnung kann funktionsbedingte Fehlfunktionen aufgrund ihrer räumlichen Lagesensitivität nicht ausschließen.

### Darstellung der Erfindung

Der Erfindung liegt die Aufgabe zugrunde eine Sprechventil-Anordnung zum Aufsetzen und Anbringen an eine Tracheostomiekanüle, mit einem einen Durchströmungskanal umschließenden Durchströmungskörper, der eine Durchströmungskörperwand aufweist, die den Durchströmungskanal radial einschließt, und der einseitig endseitig eine geeignet ausgebildete Verbindungsstruktur zur Fügung an ein proximales Ende der Tracheostomiekanüle aufweist, an dessen der Verbindungsstruktur längs des Durchströmungskanals gegenüber liegenden Ende ein Einwegventil angeordnet ist und zwischen der Verbindungsstruktur und dem Einwegventil eine durch eine Überdruckventilanordnung abschließbare Öffnung vorsieht, derart weiterzubilden, so dass die bislang bestehende Patientengefahr beim Aufsetzen eines Sprechventils auf bzw. an die Tracheostomiekanüle vermieden bzw. signifikant herabgesetzt werden kann. So soll ein vorstehend erläutertes Sprechventil zum einen sicherstellen, dass ein sich spontan aufbauender Überdruck im Luftweg eines Patienten, bspw. durch Husten, sicher und unverzüglich abgebaut wird und dies in jeder erdenklichen Raumlage der Sprechventilanordnung, d.h. unabhängig von der Einwirkungsrichtung der Schwerkraft. Alternativ oder in Kombination mit der vorstehenden Vorkehrung gilt es die Sprechventilanordnung dahingehend zu modifizieren bzw. zu ergänzen, so dass bei Auftritt einer lebensbedrohlichen Überdrucksituation, die zumindest zu einer Teilöffnung einer vorhandenen Überdruckventilanordnung führt, eine mit der Sprechventilanordnung befasste, betreuende Person über die ansonsten lebensbedrohliche Situation für den Patienten alarmiert wird.

Die Lösung der der Erfindung zugrunde liegenden Aufgabe ist im Anspruch 1 angegeben. Den Erfindungsgedanken in vorteilhafter Weise weiterbildende Merkmale sind Gegenstand der Unteransprüche sowie der weiteren Beschreibung, insbesondere unter Bezugnahme auf die Ausführungsbeispiele zu entnehmen.

Lösungsgemäß ist eine Sprechventil-Anordnung zum Aufsetzen und Anbringen an eine Tracheostomiekanüle mit den Merkmalen des Oberbegriffes des Anspruches 1 derart ausgebildet, dass die Ventilöffnung die Durchströmungskörperwand vollständig durchsetzt, und dass die Überdruckventilanordnung wenigstens eine elastisch verformbare Ventilklappe aufweist, die in einem ersten Zustand - bei dem innerhalb des Sprechventils Druckverhältnisse vorherrschen, wie sie beim Sprechen üblich sind - die zwischen der Verbindungsstruktur und dem Einwegventil am Durchströmungskörper vorgesehene Ventilöffnung abzudecken vermag und bei einem vorgebbaren Überdruck innerhalb des Durchströmungskanals durch eine Überdruck getriebene, elastische Verformung der Ventilklappe in einen die Ventilöffnung zumindest teilweise freigebenden zweiten Zustand - bei dem Überdruckbedingungen herrschen, wie sie bspw. bei Husten auftreten - überführbar ist.

Das Konstruktions- bzw. Funktionsprinzip der am Durchströmungskörper des Sprechventils angebrachten Überdruckventilanordnung ist inspiriert durch einen in der Natur vorkommenden Öffnungsmechanismus an einer bei der fleischfressenden Pflanze "Wasserschlauch" (*Utricularia vulgaris* / *Utricularia australis*) genutzten Saugfalle. Bei der Saugfalle handelt es sich um eine mit einer flexiblen Klappe verschließbaren Fangblase, die in der Lage ist, Flüssigkeit in Form von Wasser aus dem Fangblaseninneren via Drüsen in die Umgebung zu transportieren, wodurch sich die Fangblasenwände unter Ausbildung einer elastischen Wandspannung nach innen wölben, wobei der Falleneingang durch eine in diesem Zustand nach außen gewölbte Falltür, die mit ihrem freien Rand einer gewebigen Schwelle anliegt, wasserdicht verschlossen ist. In diesem Zustand herrscht im Falleninneren gegenüber dem Umgebungsdruck ein Unterdruck. Bei Kontakt mit einer Beute kehrt die Falltür ihre konvexe Krümmung innerhalb von weniger als 2 ms in eine konkave Form um, wodurch der Falleneingang zumindest bereichsweise freigegeben wird. In diesem unverschlossenen Saugfallenzustand erfolgt ein Unterdruck-getriebener Druckausgleich, wodurch sich die Saugfallenwände entspannen und Wasser sowie Beute unmittelbar vor der Saugfallenöffnung aufgrund der sich innerhalb einer Millisekunde zutragenden Volumenvergrößerung in das Innere der Saugfalle einströmen.

Die vorstehend erläuterte Funktionalität macht sich die in der Anordnung innerhalb eines Sprechventils integrierte Überdruckventilanordnung in abstrahierter (bzgl. der Druckverhältnisse umgekehrter) Weise zunutze, indem die wenigstens eine elastisch verformbare Ventilklappe, gleichsam der Fallentür im biologischen Vorbild entsprechend, ab einem schlagartig auftretenden Druckunterschied zwischen dem Inneren des Durchströmungskörpers, d.h. der Sprechventilanordnung und dem Umgebungsdruck, der üblicherweise dem aktuell vorherrschenden Luftdruck entspricht, den die Ventilöffnung zumindest teilweise freigebenden zweiten Zustand einnimmt, wodurch der Überdruck instantan abgebaut wird.

Um die Funktionalität des Sprechventils im Sprechbereich nicht zu beeinträchtigen, ist die Überdruck-abhängige Öffnung der Überdruckventilanordnung, die durch eine weitgehend verzögerungsfreie Überführung der wenigstens einen elastisch verformbaren Ventilklappe aus dem ersten in den zweiten Zustand realisiert wird, ab einem einstellbaren Druckunterschied, z.B. ab 35 bis 45 cm H2O, d.h. 0,034 bar bis 0,044 bar, zu initiieren. Im Druckbereich unterhalb des vorstehenden Auslösedruckes für die Überdruckventilanordnung, d.h. für Drucke im Sprechbereich zwischen 0 bis 25 cm H2O, d.h. 0 bis 0,0245 bar, verbleibt die wenigstens eine elastisch verformbare Ventilklappe im ersten, d.h. geschlossenen Zustand, so dass eine ausreichend druckbeaufschlagte Luftströmung im Normalbetrieb des Sprechventils die Patienten eigenen Stimmbänder zum Sprechen anzuregen vermag. Die tatsächlichen anzuwendenden Druckbereiche und die maximale Öffnungsfläche der Ventilklappe können je nach Muskelkraft, Größe und Lungenvolumen des Patienten unterschiedlich realisiert werden, indem vorzugsweise bei schwachen oder kleinen Patienten mit niedrigem Lungenvolumen der Überdruckbereich niedriger und die maximale Öffnungsfläche kleiner gewählt wird als bei kräftigen oder großen Patienten mit hohem Lungenvolumen.

Im Unterschied zu einer bekannten Kugelventilanordnung, deren Funktionsweise durch die Eigenschwere der Kugel und damit verbunden durch die räumliche Anordnung und Lage des gesamten Kugelventils abhängt, spielt die Eigenschwere der wenigstens einen elastisch verformbaren Ventilklappe für die Funktionsweise der lösungsgemäßen Überdruckventilanordnung keine bzw. keine nennenswerte Rolle, wodurch die Funktionsweise der lösungsgemäßen Anordnung innerhalb des Sprechventils in jeder räumlichen Anordnung und Lage gewährleistet bleibt.

Der Durchströmungskörper weist eine den Durchströmungskanal radial umfassende Durchströmungskanalwand auf, die lokal von der Ventilöffnung der Überdruckventilanordnung durchsetzt ist. Die Ventilöffnung ist längs des Durchströmungskanals beabstandet angeordnet, vorzugsweise mittig, zwischen der Verbindungsstruktur und dem Einwegventil. Der Ventilöffnung ist eine Öffnungsnormale zuordenbar, die orthogonal zur Kanallängsachse des Durchströmungskanals orientiert ist, d.h. die Ventilöffnung ist der Kanallängsachse zugewandt orientiert.

In einer bevorzugten Ausführungsform ist eine einzige elastisch verformbare, flächig ausgebildete Ventilklappe vorgesehen, die im ersten Zustand die Ventilöffnung abdeckt, wobei die elastisch verformbare Ventilklappe relativ zur Ventilöffnung derart mittel- oder unmittelbar an den Durchströmungskörper gefügt ist, so dass die elastisch verformbare Ventilklappe den Durchströmungskanal zu- und der Ventilöffnung abgewandt angeordnet ist. In einer alternativen Ausführungsform ist die Ventilklappe außerhalb des Durchströmungskörpers angeordnet und verschließt die Ventilöffnung im ersten Zustand rückseitig. Auf diese Weise ist sichergestellt, dass in einem Schadensfall die Ventilklappe oder auch nur Teile davon nicht in den patientenseitigen Atemweg gelangen können. Zudem eröffnet sich auf diese Weise die Ventilklappe in Form eines Austauschmoduls sicher und schnell auszutauschen, ohne dabei das Innere des Sprechventils öffnen zu müssen.

In allen möglichen Ausführungsbeispielen sind der Ventilöffnung zwei Halbräume zuordenbar, von denen in einem der beiden Halbräume die elastisch verformbare Ventilklappe wenigstens abschnittsweise ortsfest relativ zur Ventilöffnung gefügt ist und innerhalb des anderen der beiden Halbräume die elastisch verformbare Ventilklappe lose zur Ventilöffnung gelagert ist. Im übertragenen Sinne entspricht die Fügung der elastisch verformbaren Ventilklappe relativ zur Ventilöffnung einer einseitig an einer Türzarge drehbeweglichen Anbringung einer Schwenktür. Im Unterschied zur Anbringung und Ausbildung einer an sich bekannten Schwenktür deckt die wenigstens eine elastisch verformbare Ventilklappe im ersten Zustand die Ventilöffnung innerhalb ihres zweiten Halbraumes zumindest abschnittsweise mit einem randseitigen Übermaß ab, d.h. die elastisch verformbare Ventilklappe erstreckt sich zumindest abschnittsweise über einen Bereich des Ventilöffnungsrandes hinaus. Dieses randseitige Übermaß trägt dazu bei, dass die elastisch verformbare Ventilklappe bei einem in Betrieb befindlichen Sprechventil so lange innerhalb des ersten Zustandes, d.h. in dem die Ventilöffnung verschließenden Zustand verbleibt, solange der innerhalb der Sprechventilanordnung vorherrschende Druck unterhalb des vorgegebenen Überdruckbereichs, verbleibt.

Neben der Dimensionierung des Übermaßes spielen eine Vielzahl weiterer Material- und Designparameter eine wichtige Rolle zur Einstellung der Überdruck-getriebenen Öffnungsfunktion der neuartig ausgebildeten Überdruckventilanordnung. Wie im weiteren unter Bezugnahme auf illustrierte Ausführungsbeispiele erläutert wird, tragen Formgebung, Größe und Materialwahl für die Ausbildung der wenigstens einen elastisch verformbaren Ventilklappe sowie auch die Geometrie der randseitigen Ventilöffnungskontur für eine sichere und zuverlässige Funktion der Überdruckventilanordnung bei.

Vorzugsweise ist das randseitige Übermaß nicht gleich bemessen, sondern weist an wenigstens einer Stelle ein Minimum auf. Auf diese Weise lässt sich der Öffnungsvorgang durch die Überdruck-getriebene elastische Verformung der wenigstens eine Ventilklappe in einer geometrisch vorbestimmten Weise festlegen, in der die wenigstens eine Ventilklappe zeitlich am schnellsten eine elastische Umformung vom ersten in den zweiten Zustand realisiert.

Aufgrund der typischerweise zylinderförmigen Ausgestaltung des Durchströmungskörpers an sich bekannter Sprechventilaufsätze auf Tracheostomiekanülen bietet es sich an, die Ventilöffnung, vorzugsweise mittig, beabstandet zwischen der Verbindungsstruktur und dem Einwegventil durch eine rechteckförmige oder schlitzförmige Ausnehmung innerhalb der Durchströmungskörperwand auszubilden oder durch eine Ventilöffnung, die sich an der rechteckförmigen oder schlitzförmigen Ausnehmung innerhalb der Durchströmungskörperwand anschließt und deren größere Öffnungsdimension in Umfangsrichtung um den Durchströmungskanal und deren kleinere Öffnungsdimension parallel zum Durchströmungskanal angeordnet sind.

Dabei ist die wenigstens eine elastisch verformbare Ventilklappe flächig ausgebildet und verfügt über eine flächig gebogene Form entsprechend angepasst zur Abdeckung der Ventilöffnung.

Um dem bereits erwähnten Aspekt der unterschiedlichen Sprechdrucke bei Patienten unterschiedlicher Konstitution Rechnung zu tragen, ist die wenigstens eine elastisch verformbare Ventilklappe austauschbar mittel- oder unmittelbar am Durchströmungskörper der Anordnung in Art eines Sprechventils anbringbar. Zur lösbar einseitig festen Anbringung der elastisch verformbaren Ventilklappe dienen in einer Ausführungsvariante zwei Befestigungspins, an denen die Ventilklappe unter Ausbildung einer flächig in sich gebogenen Raumform aufsteckbar ist. In diesem Fall sind die zwei Befestigungspins dem Durchströmungskanal zugewandt im Bereich der Überdruckventilanordnung angebracht. In einem alternativen Ausführungsbeispiel schließt die Überdruckventilanordnung im Bereich der Ventilöffnung außerhalb des Durchströmungskörpers an und sieht eine schlitzförmige Einführöffnung vor, in welche die Ventilklappe in Form eines Einschubmoduls einsetzbar ist. Die Ventilklappe kann in dieser Ausführungsform aus Hygienegründen von außen gewechselt werden, ohne dabei das Sprechventil öffnen zu müssen. Hierzu wird eine Halterung zur lösbar festen Fixierung der Ventilklappe entfernt, die sodann aus der schlitzförmigen Einführöffnung herausgezogen und durch eine Neue ersetzt werden kann. Ein unbeabsichtigtes Abrutschen und Einatmen der Ventilklappe wird durch die Halterung sowie das standardmäßige Partikelfilterschaumelement im Sprechventil verhindert.

Durch Bevorratung einer Anzahl von Ventilklappen, die sich wenigstens durch ihre Form, Größe oder ihren elastischen Eigenschaften voneinander unterscheiden, kann eine patientenindividuelle Auswahl getroffen werden, um den Auslösedruck der Überdruckventilanordnung Patienten-spezifisch wählen zu können. Nähere Einzelheiten können im Weiteren der Beschreibung unter Bezugnahme auf ein illustriertes Ausführungsbeispiel entnommen werden.

Alternativ oder in Kombination zur Ausbildung der vorstehend erläuterten sicher und Lage-unabhängig öffnenden Überdruckventilanordnung lässt sich die bei Fehlbedienung einer Sprechventilanordnung entstehende Lebensgefahr für den Patienten dadurch vermeiden bzw. signifikant reduzieren, indem mittel- oder unmittelbar an der Überdruckventilanordnung oder stromab zu dieser, wie auch immer die Überdruckventilanordnung ausgebildet sein mag, ein Signalerzeugungsmittel angeordnet ist, das im Falle eines Überdruck-getriebenen Öffnens der Überdruckventilanordnung, d.h. im Überdruckbereich, ein Signal erzeugt.

Vorzugsweise ist das Signalerzeugungsmittel zur Erzeugung eines akustisch wahrnehmbaren Schallsignals ausgebildet, das von einer die Sprechventilanordnung handhabenden Person in Form eines Alarmsignals unüberhörbar wahrgenommen werden kann. Durch das Alarmsignal wird die Person auf einen etwaigen Fehler aufmerksam gemacht, so dass der Fehler einer geblockten Tracheostomiekanüle unverzüglich korrigiert werden kann.

Gleichzeitig zur Signalerzeugung erfolgt eine spontane Druckentlastung durch die geöffnete Überdruckventilanordnung, wodurch die Gefahr des sofortigen Erstickens und Platzens der Patientenlunge verhindert wird. Der den Sprechventilaufsatz handhabenden Person wird dadurch Zeit gegeben, den Bedienfehler zu korrigieren. Durch die Kombination einer Signalerzeugung mit der Funktion der Druckentlastung, vorzugsweise auf Basis der vorstehend erläuterten, lösungsgemäß ausgebildeten Überdruckventilanordnung, werden einerseits die ungestörte Funktion des Sprechventils im normalen Einsatz, d.h. bei normalen Atmen und Sprechen vorherrschenden Drucken (Sprechbereich), ohne Fehlalarme gewährleistet und andererseits bei Überschreiten eines vorgegebenen Überdruckbereichs innerhalb des Sprechventilaufsatzes sowohl eine Signalauslösung als auch eine unverzügliche Druckentlastung zum Schutze des Patienten gleichzeitig realisiert. Reduziert sich nach Auslösen, d.h. Öffnen der Überdruckventilanordnung, der im Durchströmungskörper vorherrschende Druck, d.h. bspw. durch Einatmen, so schwingt die elastische Ventilklappe in ihre Ausgangssituation zurück und verschließt die Ventilöffnung selbstständig, so dass das Sprechventil in gewohnter Weise weiter benutzt werden kann.

In einer bevorzugten Ausführungsform ist das Signalerzeugungsmittel in Art einer Pfeife ausgebildet und befindet sich innerhalb eines sich stromab an die Überdruckventilanordnung anschließenden Strömungskanals. Je nach Ausbildung und Anordnung der Pfeife bietet es sich an, den Strömungskanal unter Ausbildung einer Labial- oder Lingualpfeife lokal zu strukturieren.

Alternativ oder in Kombination vermag das Signalerzeugungsmittel ein elektrisches, elektromagnetisches oder optisches Signal zu erzeugen, das entweder in unmittelbarer Umgebung zur physiologischen Wahrnehmung durch eine Person abgegeben wird oder mittels Kabel bzw. kabellos an eine Überwachungseinheit, beispielsweise in Form einer zentralen Monitoreinheit, übertragen wird. Hierzu ist eine die Überdrucksituation erfassende Sensorik in oder an die Überdruckventilanordnung anzuordnen, deren Sensorsignale einer zentralen Überwachungseinheit zur Alarmauslösung zuführbar oder in eine physiologisch wahrnehmbare Form transformierbar sind. Die Sensorik kann dabei mit dem Ventilmechanismus kombiniert sein oder als unabhängiger Signalgeber realisiert werden.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnung exemplarisch beschrieben. Es zeigen:
- Fig. 1: perspektivische Längsschnittdarstellung durch eine lösungsgemäß ausgebildete erste Anordnung in Art eines Sprechventils,
- Fig. 2a-f: Querschnittsdarstellung der lösungsgemäßen ersten Anordnung einer Überdruckventilanordnung als Sequenzbilder,
- Fig. 3a-j: perspektivische Längsschnittbilder durch eine lösungsgemäß ausgebildete erste Anordnung in Art eines Sprechventils als Sequenzbilder
- Fig. 4: perspektivische Querschnittsdarstellung durch die lösungsgemäß ausgebildete erste Anordnung in Art eines Sprechventils,
- Fig. 5: perspektivische Längsschnittdarstellung durch eine lösungsgemäß ausgebildete zweite Anordnung in Art eines Sprechventils,

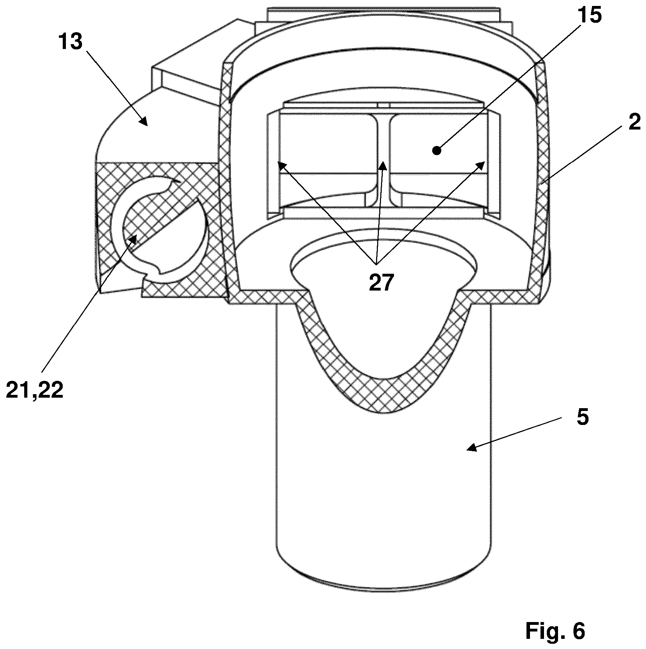

- Fig. 6: 90° zu Fig. 5 verdrehte perspektivische Längsschnittdarstellung durch die lösungsgemäß ausgebildete zweite Anordnung in Art eines Sprechventils,

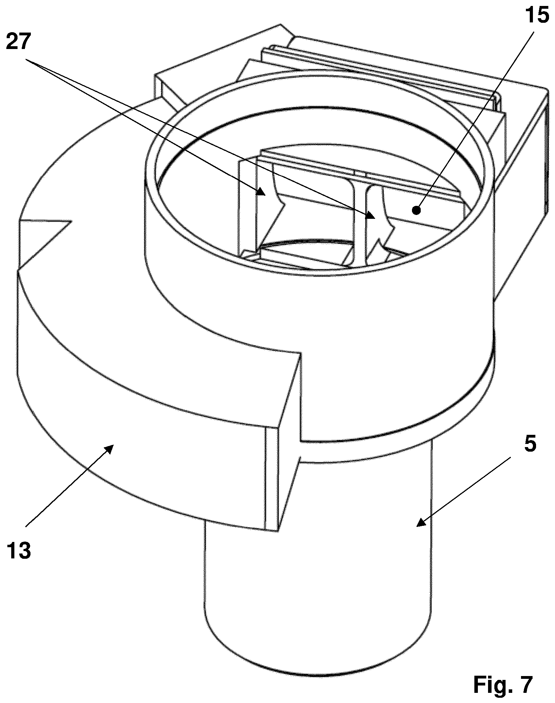

- Fig. 7: perspektivische Schrägansicht auf die lösungsgemäß ausgebildete zweite Anordnung in Art eines Sprechventils ohne Einwegventil, sowie
- Fig. 8a-f: perspektivische Längsschnittbilder durch die Überdruckventilanordnung der lösungsgemäß ausgebildeten zweiten Anordnung in Art eines Sprechventils in Form von Sequenzbildern.

### Wege zur Ausführung der Erfindung, gewerbliche Verwendbarkeit

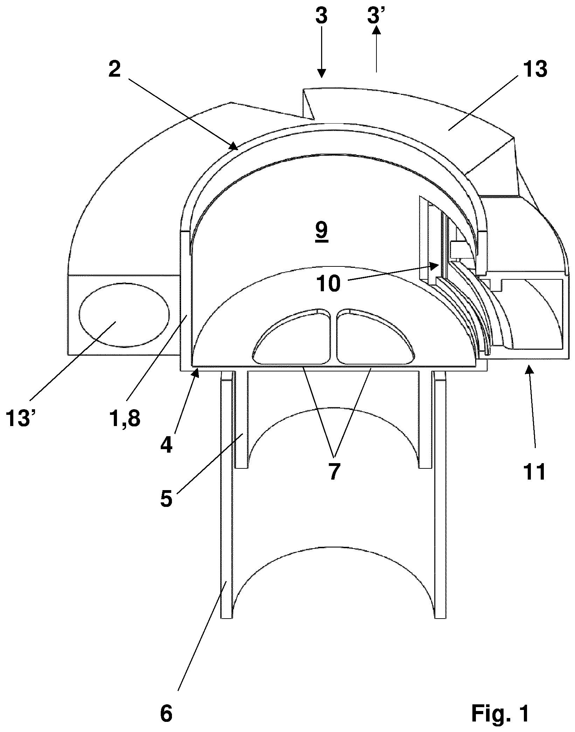

Figur 1 zeigt eine perspektivische Längsschnittdarstellung durch eine Sprechventilanordnung, die über einen hohlzylinderförmigen Durchströmungskörper 1 verfügt, an dessen oberen, in Figur 1 aus besseren Darstellungsgründen offen ausgebildeten Durchströmungskörperende 2 ein nicht weiter, an sich bekanntes Einwegmembranklappenventil angeordnet ist, das das obere Durchströmungskörperende für eine inspiratorische Luftzuströmung 3 öffnet und eine respiratorische Luftabströmung 3' unterbindet.

An dem in Figur 1 unteren Durchströmungskörperende 4 schließt eine Verbindungsstruktur 5 an, über die die Sprechventilanordnung vorzugsweise abnehmbar fluiddicht an eine Tracheostomiekanüle 6 fügbar ist. Am Übergang zwischen Durchströmungskörper 1 und Verbindungsstruktur 5, die im gezeigten Ausführungsbeispiel über einen geringeren Innendurchmesser verfügt als der Durchströmungskörper 1, ist eine Gitterstruktur 7 angeordnet, die zum einen eine Schutzfunktion gegen unkontrollierten Eintritt von Fremdpartikeln in Richtung der Tracheostomiekanüle 6 besitzt und zum anderen eine mechanisch stabile Auflageebene für ein nicht dargestelltes, standardmäßiges Partikelfilterschaumelement bietet.

Zusätzlich sieht die Anordnung in Art eines Sprechventils innerhalb des Durchströmungskörpers 1, dessen Durchströmungskörperwand 8 einen Durchströmungskanal 9 umfasst, eine Öffnung 10 vor, die die Durchströmungskörperwand 8 vollständig durchsetzt und zwischen dem oberen Durchströmungskörperende 2 und dem unteren Durchströmungskörperende 4 angeordnet ist.

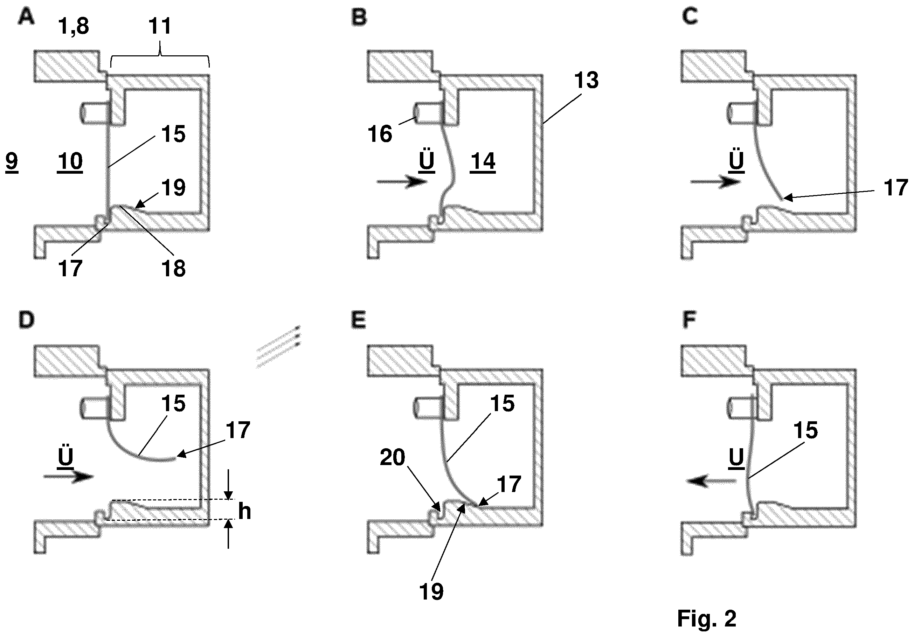

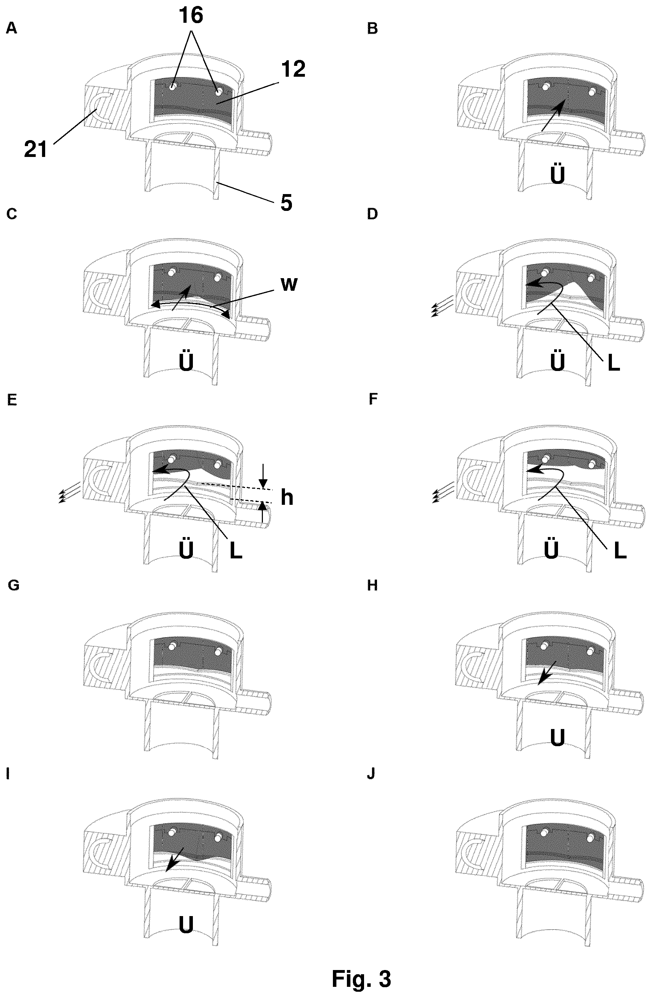

An der Öffnung 10 ist eine Überdruckventilanordnung 11 angebracht, deren Aufbau und Funktionsweise unter Bezugnahme der in den Figuren 2 und 3 entnehmbaren Sequenzbildern im Weiteren näher erläutert wird.

Die Sequenzbilder der Figuren 2a bis f stellen jeweils Längsschnitte durch die an der Öffnung 10 innerhalb der Durchströmungskörperwand 8 des Durchströmungskörpers 1 angeordneten Überdruckventilanordnung 11 dar. Die Durchströmungskörperwand 8 umfasst radial den Durchströmungskanal 9. Die in den Figuren 3a bis j gezeigten Sequenzbilder stellen perspektivische Längsschnitte durch die gesamte Anordnung in Art eines Sprechventils dar. Sämtliche in den Figuren 2 und 3 illustrierten Sequenzbilder vermögen die Funktionsweise und das dynamische Verformungsverhalten der die Überdruckventilfunktion bestimmenden elastisch verformbaren Ventilklappe 15 nachvollziehbar zu charakterisieren.

Die Überdruckventilanordnung 11 schließt sich unmittelbar radial außerhalb des Durchströmungskörpers 1 an die Öffnung 10 innerhalb der Durchströmungskörperwand 8 fluiddicht an. Die Überdruckventilanordnung 11 kann als separate Baueinheit zum Durchströmungskörper 1, wie dargestellt, ausgebildet sein oder einstückig mit diesem verbunden sein. Die Überdruckventilanordnung 11 verfügt über eine Ventilöffnung 12, die im dargestellten Fall kleiner dimensioniert ist als die die Durchströmungskörperwand 8 durchragende Öffnung 10. Denkbar sind jedoch auch Ausführungsvarianten für die Überdruckventilanordnung 11, in denen die Öffnung 10 sowie die Ventilöffnung 12 identisch sind.

Die Überdruckventilanordnung 11 weist eine, einen Strömungskanal 14 umfassende Strömungskanalwand 13 auf, die über wenigstens eine Öffnung 13' in die Umgebung mündet, s. Fig. 1. Somit herrschen innerhalb des Strömungskanals 14 Umgebungsdruckbedingungen.

Die Ventilöffnung 12 ist in einem ersten Zustand, siehe Figur 2a, 3a von einer elastisch verformbaren Ventilklappe 15 vollständig abgedeckt. Die Ventilklappe 15 ist membran- bzw. folienartig ausgebildet und besteht bspw. aus einem Elastomer. Zur Anbringung der Ventilklappe 15 innerhalb der Überdruckventilanordnung 11 dienen zwei Befestigungspins 16 die in radialer Projektion auf die Öffnung 10 innerhalb des Öffnungsbereiches der Öffnung 10 an der Überdruckventilanordnung 11 angebracht sind, siehe insbesondere die Darstellung gemäß Figur 3a bis j. Die elastisch verformbare Ventilklappe 15 weist zu den Befestigungspins 16 korrespondierende Löcher auf, so dass die Ventilklappe 15 längs ihres oberen Ventilklappenrandes abnehmbar fest an der Überdruckventilanordnung 11 gefügt ist.

Form und Größe der elastisch verformbaren Ventilklappe 15 sind an die Form und Größe der Ventilöffnung 12 derart angepasst, so dass sie axial beiderseits die Ventilöffnung 12 mit einem Übermaß überragt, wohingegen die Erstreckung der Ventilklappe 15 in Umfangsrichtung maximal der Ventilöffnungsweite w der Ventilöffnung 12 entspricht, s. Figur 3c. Die Fügung der elastisch verformbaren Ventilklappe 15 an die Überdruckventilanordnung 11 gestattet überdies einen unkomplizierten Austausch der Ventilklappe 15.

In einem nicht gefügten Zustand ist die elastisch verformbare Ventilklappe 15 flächig rechteckförmig eben ausgebildet. Nach Fügen der Ventilklappe 15 in die Überdruckventilanordnung 11 mittels der Befestigungspins 16 wird die Ventilklappe 15 in einen flächig gebogenen, entsprechend angepasst an die zylindrische Krümmung der Durchströmungskörperwand 8 überführt, wodurch die elastisch verformbare Ventilklappe 15 eine durch die Biegung, deren Größe, Form und Eigenelastizität des Ventilklappenmaterials mitbestimmte Formstabilität erhält.

Der den Befestigungspins 16 axial gegenüberliegende untere Ventilklappenrand 17 der Ventilklappe 15 überragt axial den die Ventilöffnung 12 begrenzenden unteren Rand, der schwellenartig ausgebildet ist, siehe hierzu Figuren 2a bis f. Die schwellenartige Ausbildung des unteren Ventilöffnungsrandes 18 sieht eine der Ventilklappe 15 im ersten Zustand zugewandte axial orientierte Flanke vor, an der der untere Ventilklappenrand 17 mit Übermaß bündig anliegt. In Richtung des inneren Strömungskanals 14 der Überdruckventilanordnung 11 schließt sich eine kontinuierlich abfallende Schwellenflanke 19 an, deren Funktion im Weiteren noch erläutert wird. Zudem verfügt der schwellenförmig ausgebildete untere Ventilöffnungsrand 18 in Umfangsrichtung der Überdruckventilanordnung 11 über eine variierende Schwellenhöhe h dergestalt, dass die Schwellenhöhe h jeweils an beiden sich in Umfangsrichtung gegenüberliegenden Ventilöffnungsrändern maximal und jeweils mittig minimal ist, siehe Figuren 2d, 3e,f. Auf diese Weise überlappt die elastisch verformbare Ventilklappe 15 die Ventilöffnung 12 jeweils mit einem maximalen Übermaß an ihren Randbereichen und mit einem minimalen Übermaß im mittigen Bereich der Ventilklappe 15.

Die Form, Größe, Dicke sowie das elastomere Material der elastisch verformbaren Ventilklappe 15 bestimmen das elastisch Verformungsverhalten der Ventilklappe und legen somit den für das Öffnen der Überdruckventilanordnung erforderlichen Mindestüberdruck fest. Durch Vorsehen einer Anzahl unterschiedlich konfektionierter Ventilklappen, die sich in ihren elastischen Verformungsverhalten voneinander unterscheiden, kann die lösungsgemäße Anordnung in Art eines Sprechventils an die Beatmungsphysiologischen Fähigkeiten bzw. Eigenschaften eines Patienten individuell angepasst werden.

Im Weiteren wird auf die Sequenzbilder sowohl der Figuren 2 und 3 gemeinsam Bezug genommen, um die Überführung der elastisch verformbaren Ventilklappe 15 aus dem ersten Zustand, in dem die Überdruckventilanordnung 11 geschlossen ist, in den zweiten Zustand, in dem die Überdruckventilanordnung 11 eine geöffnete Stellung einnimmt, näher zu erläutern.

Die Figuren 2a, 3b zeigen die Überdruckventilanordnung 11 im ersten Zustand, d.h. die Ventilklappe 15 schließt die Ventilöffnung 12 weitgehend gasdicht ab und sorgt dafür, dass sich innerhalb des Durchströmungskörpers 1 Druckverhältnisse ausbilden können, die dem Patienten ein Sprechen ermöglichen.

Erhöht sich der Druck innerhalb des Durchströmungskörpers 1, beispielsweise durch spontanes Husten, so wird die elastisch verformbare Ventilklappe 15 Überdruck-getrieben deformiert, siehe Figur 2b, 3b. Bei einem ausreichend hohen, innenseitig, d.h. von Seiten des Durchströmungskanals 9, auf die Ventilklappe 15 lastenden Überdruckes Ü wird die Ventilklappe 15 durch das Druckereignis vornehmlich im mittigen Bereich radial nach außen deformiert, so dass der untere frei bewegliche Ventilklappenrand 17 den unteren schwellenartig ausgebildeten Ventilöffnungsrand 18, der mittig gegenüber den Randbereichen abgesenkt ist, überstreift. In diesem sich öffnenden Moment der Überdruckventilanordnung 11 bildet sich aufgrund der schwellenartigen Geometrie längs des unteren Ventilöffnungsrandes 18 eine zentrale senkrechte Falz am unteren Ventilklappenrand 17 aus, siehe Figur 3c, wodurch sich die Ventilklappe 15 von ihrer Mitte ausgehend schlag- bzw. sprungartig radial nach außen in das Volumen der Überdruckventilanordnung 11 deformiert, siehe Figur 3d, 2c. In diesem Moment kommt es sowohl zu einer temporären Versteifung der Ventilklappe 15 in ihrem Zentrum als auch zu einer strukturellen Instabilität der Fläche der Ventilklappe 15 durch das Aufheben ihrer Flächenkrümmung über die gesamte Ventilklappe 15. Im Weiteren wird die Ventilklappe 15 Überdruck-getrieben von ihrer Mitte ausgehend in das Lumen, bzw. in den Strömungskanal 14 der Überdruckventilanordnung 11 verdrängt, wobei die randseitigen Bereiche der Ventilklappe vom unteren Ventilöffnungsrand 18 nachgezogen werden. Die Ventilöffnung 12 wird freigegeben, siehe die Figuren 2c, 2d bzw. 3e, f, wobei der sich schlagartig innerhalb des Durchströmungskörpers 1 ausgebildete Überdruck Ü durch eine durch die Ventilöffnung 12 gerichtete Luftströmung L effektiv abgebaut wird.

Durch Abbau des Überdruckes bzw. vollständigen Druckausgleich stellt sich die elastisch verformbare Ventilklappe aufgrund ihrer Eigenelastizität wieder zurück, siehe Figur 2e, 3g und kommt mit ihrem unteren Ventilklappenrand 17 zur Anlage an die schräg geneigte Schwellenflanke 19 des unteren Ventilöffnungsrandes 18, siehe Figur 2e. Erfolgt kein weiterer Druckstoß, ausgelöst durch Husten, wird die Ventilklappe 15, gegebenenfalls unterstützt durch einen sich innerhalb des Durchströmungskörpers 1 einatmungsbedingt ausbildenden Unterdrucks U in den ersten Zustand rückgeführt, in dem die Ventilklappe 15 die Ventilöffnung 12 vollständig abdeckt, siehe Figur 2f, 3j.

In einer bevorzugten Ausführungsform, wie sie in den Sequenzbilddarstellungen gemäß Figur 2a bis f gezeigt ist, mündet der untere Ventilklappenrand 17 in eine nutförmige Ausnehmung 20, wodurch zusätzlich zur krümmungsbedingten Eigenstabilität der Ventilklappe 15 im ersten Zustand die Bewegungsmöglichkeit der Ventilklappe 15 begrenzt ist. Sowohl durch die gebogene Form der Ventilklappe 15, die durch die zylindrische bzw. runde Form des Durchströmungskörpers bedingt ist, sowie durch ein auf der Gitterstruktur 7 aufliegenden Partikelfilterschaumelementes (nicht dargestellt) sowie durch die Fügung der Ventilkappe 15 innerhalb der nutförmigen Ausnehmung 20 wird ein Durchschlagen oder Einsaugen der Ventilklappe 15 in das Innere der Sprechventilanordnung verhindert.

Das aus dem Bereich der Biologie entlehnte Ventilklappenprinzip, wie eingangs erläutert, basiert ausschließlich auf einer isotrop wirkenden Druckdifferenz zwischen dem Inneren des Durchströmungskörpers 1 und der Umgebung. Somit ist die Funktionsweise der "bionisch angelehnten" Überdruckventilanordnung 11 lageunabhängig.

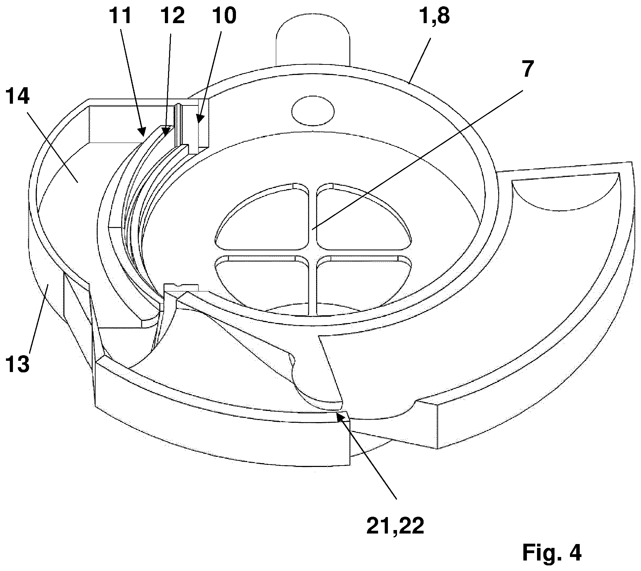

In einer bevorzugten Weiterbildung der Anordnung nach Art eines Sprechventils, zur Begegnung der Gefahr einer für den Patienten lebensbedrohlichen Situation durch unsachgemäße Handhabung der Tracheostomiekanüle, ist stromab zur Überdruckventilanordnung 11 ein Signalerzeugungsmittel 21 zur Erzeugung eines akustisch wahrnehmbaren Schallsignals, vorzugsweise in Form einer Pfeife 22, angeordnet, siehe hierzu Figur 3 sowie Figur 4. In Figur 4 ist der Durchströmungskörper 1 samt Überdruckventilanordnung 11 in einem Querschnitt dargestellt. Stromab zur Öffnung 10 bzw. Ventilöffnung 12 schließt sich ein Strömungskanal 14 an, der im Falle der in Figur 4 dargestellten Ausführungsform in Umfangsrichtung des Durchströmungskörpers 1 orientiert ist. Längs des Durchströmungskanals 12 ist eine Pfeife 21, vorzugsweise in Form einer Labial-Pfeifenkontur angeordnet. Kommt es zu einer Patienten-gefährdenden Überdrucksituation, so wird die Überdruckventilanordnung 11, beispielsweise in der vorstehend genannten Weise, schlagartig geöffnet, wodurch ein Entweichen der Überdruck-getriebenen Luftströmung durch die Überdruckventilanordnung 11 im Bereich der Pfeife 22 einen für das Umfeld alarmierenden akustisch wahrnehmbaren Signalton erzeugt, so dass eine den Patienten betreuende Person unverzüglich auf die Gefahrensituation aufmerksam gemacht wird und entsprechende Gegenmaßnahmen treffen kann.

Nicht notwendigerweise jedoch in vorteilhafter Form ist das Signalerzeugungsmittel 21 mit dem vorstehend erläuterten bionischen Überdruckventil in der in Figur 4 entnehmbaren Weise kombiniert. Gleichsam können an sich bekannte Überdruckventilanordnungen, beispielsweise in Form eines Kugelventils, im Bereich der Luftauslassöffnung mit einem entsprechenden Signalerzeugungsmittel, vorzugsweise in Form einer Pfeife, versehen sein.

Denkbar ist auch die Ausgestaltung des Signalerzeugungsmittels zur Erzeugung eines elektrischen, elektromagnetischen oder optischen Signals, das gegebenenfalls mittels Kabel oder kabellos an eine zentrale Überwachungseinheit übertragbar ist. Hierzu stehen dem Fachmann an sich bekannte Sensorsysteme zur Verfügung, mit denen bspw. eine Auslenkung der elastisch verformbaren Ventilkappe und/oder eine Luftströmung innerhalb des Strömungskanals detektierbar ist.

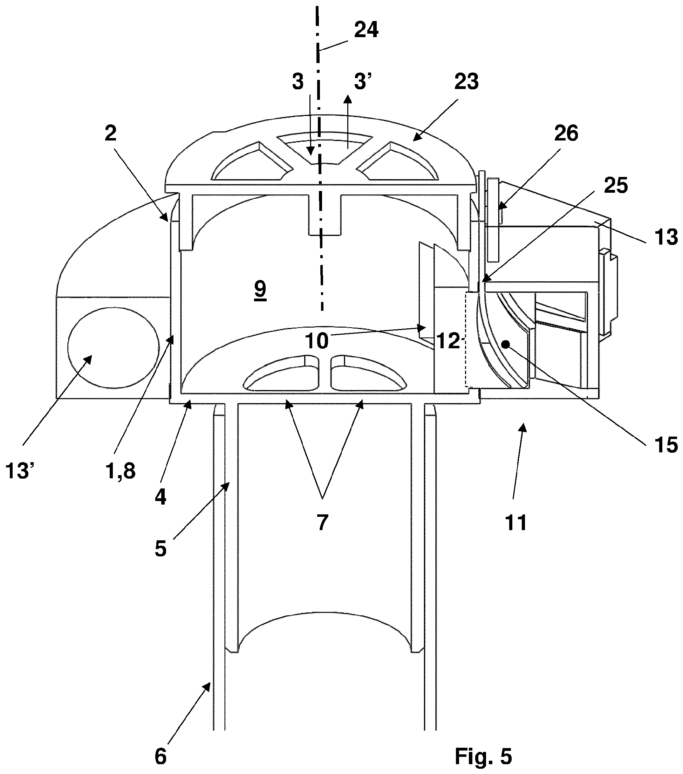

Figur 5 zeigt ein zweites Ausführungsbeispiel für eine lösungsgemäße Sprechventilanordnung, die gleichsam wie jene in Figur 1 in perspektivischer Längsschnittdarstellung gezeigt ist. Um Wiederholungen zu vermeiden, entsprechen die bereits erläuterten und auch im weiteren verwendeten Bezugszeichen jenen vorstehend beschriebenen Komponenten, deren Funktion nicht erneut erläutert werden. In Ergänzung zu Figur 1 ist in Figur 5, das am oberen, offen ausgebildeten Durchströmungskörperende 2 ein sich bekanntes Einwegmembranklappenventil angeordnet, das das obere Durchströmungskörperende 2 für eine inspiratorische Luftzuströmung 3 öffnet und für eine respiratorische Luftabströmung 3' unterbindet.

Zusätzlich sieht die Anordnung in Art eines Sprechventils innerhalb des Durchströmungskörpers 1, dessen Durchströmungskörperwand 8 einen Durchströmungskanal 9 umfasst, eine Öffnung 10 vor, die die Durchströmungskörperwand 8 vollständig durchsetzt und zwischen dem oberen Durchströmungskörperende 2 und dem unteren Durchströmungskörperende 4 angeordnet ist, d.h. die Öffnung 10 ist längs des Durchströmungskanals 9 jeweils beabstandet zum oberen und unteren Durchströmungskörperende 2, 4 angeordnet ist dem Durchströmungskanal 9 quer zu dessen Längserstreckung 24 zugewandt orientiert.

An der Öffnung 10 schließt außerhalb der Durchströmungskörperwand 8 eine Überdruckventilanordnung 11 an, deren Aufbau und Funktionsweise grundsätzlich jener in den Figuren 1 bis 4 illustrierten Überdruckventilanordnung 11 gleicht. Im Unterschied zu dieser ist jedoch die Ventilklappe 15 (Figur 5) nicht dem Durchströmungskanal 9 zugewandt und an zwei Befestigungspins 16 lösbar befestigt, wie aus Fig. 2 A bis F sowie Fig. 3 A bis J ersichtlich, vielmehr ist die Ventilklappe 15 in Form eines Einsteckmoduls in eine schlitzförmige Ausnehmung 25 innerhalb der Strömungskanalwand 13 der Überdruckventilanordnung 11 fügbar und mittels einer Halterung 26 an der Außenseite der Durchströmungskörperwand 8 fixierbar. Auf diese Weise vermag die Ventilklappe 15 im ersten Zustand, bei dem sich innerhalb des Durchströmungskörpers 1 Druckverhältnisse ausbilden, die dem Patienten ein Sprechen ermöglichen, die unmittelbar radial nach außen an die die Durchströmungskörperwand 8 durchragende Öffnung 10 anschließende Ventilöffnung 12 der Überdruckventilanordnung 11 von Seiten der dem Durchströmungskanal abgewandten Seite abzudecken bzw. zu verschließen. Durch die außerhalb des Durchströmungskörpers 1 vorgenommene Anbringung der Ventilklappe 15 ist es ausgeschlossen, dass sich die Ventilklappe 15 unkontrolliert ablösen und in den Durchströmungskanal 9 gelangen kann.

Zudem sieht die Überdruckventilanordnung 11 mittig und randseitig zur Ventilöffnung 12 jeweils gebogen ausgeführte Kulissenwände 27 vor, an die sich die Ventilklappe 15 im ersten Zustand anschmiegt und auf diese Weise eine definiert vorgegebene Ventilklappenkrümmung einnimmt, die, wie bei dem biologischen Vorbild dem Falltürsystem der Saugfalle des Wasserschlauchs (Gattung *Utricularia*) - wie eingangs erläutert - zur Seite des einwirkenden Drucks, d.h. dem Sprechventilkorpus zugewandt, konvex gebogen ist. Der Radius dieser Krümmung ist durch die Kulissenwände festgelegt.

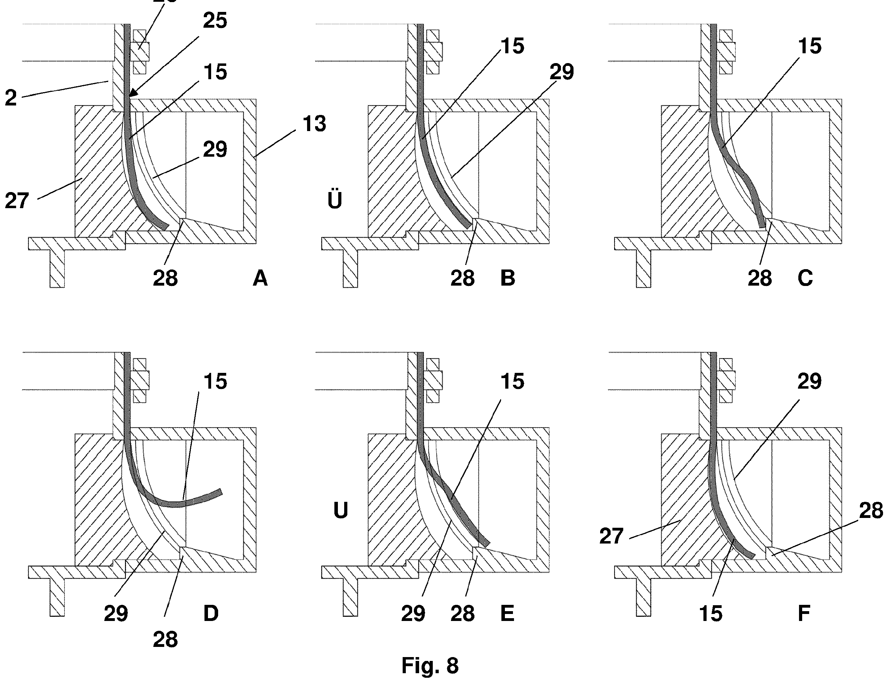

Im Weiteren wird auf die Sequenzbilder A bis F der Figur 8 Bezug genommen, um die Überführung der elastisch verformbaren Ventilklappe 15 aus dem ersten Zustand, in dem die Überdruckventilanordnung 11 geschlossen ist, in den zweiten Zustand, in dem die Überdruckventilanordnung 11 eine geöffnete Stellung einnimmt, und zurück, näher zu erläutern.

Im Ruhezustand, siehe Figur 8 A, liegt die Ventilklappe 15 auf den Kulissenwänden 27 auf. Am unteren Rand der Ventilöffnung 12 ist zudem eine Schwellenstruktur 28 vorgesehen. An den beiden, die Ventilöffnung 12 seitlich begrenzenden Seitenwänden sind jeweils seitliche Schwellenstrukturen 29 angebracht. Im Falle des normalen Sprechens wird die Ventilklappe 15 aus ihrer Ruhelage ausgelenkt, siehe Figur 8 B, sodass die untere Kante der Ventilklappe 15, wie auch beim biologischen Vorbild, an der Innenseite der unteren Schwellenstruktur 28 anliegt. Um eine Leckageströmung in dieser geschlossenen Ventilstellung zu verhindern, liegen zudem beide Seitenkanten der Ventilklappe 15 von Innen an den seitlichen Schwellenstrukturen 29 dicht an.

Bei einem schlagartigen Druckereignis innerhalb des Sprechventilkorpus, bspw. initiiert durch Husten, kommt es zu einer Krümmungsänderung der Ventilklappe 15, vorzugsweise in der Mitte der flächigen Ventilklappe 15, siehe Figur 8 C, woraufhin die Ventilklappe 15 über die Schwellenstrukturen 28 und 29 durchschlägt und die Ventilöffnung 12 nachfolgend vollständig öffnet, siehe Figur 8 D.

Bei einer nachfolgenden Inspiration durch den Patienten entsteht im Sprechventilinneren ein Unterdruck (U), wodurch die Ventilklappe 15 an den Schwellenstrukturen 28, 29 rückseitig abdichtet, siehe Figur 8 E, um nachfolgend wieder in ihre Ausgangsposition zurückkehren, siehe Figur 8 F. Um die Rückstellung Inspirations-getrieben sicherzustellen, sind die Schwellenstrukturen 28, 29 an den im Ausgangszustand Ventilklappen-abgewandten Schwellenseite jeweils abgeflacht.

Stromab zur Überdruckventilanordnung 11 ist, wie auch bei der Sprechventilanordnung gemäß dem in den Figuren 1 bis 4 illustrierten Ausführungsbeispiel, ein Signalerzeugungsmittel 21 zur Erzeugung eines akustisch wahrnehmbaren Schallsignals, vorzugsweise in Form einer Pfeife 22, angeordnet, so dass zur diesbezüglichen Erläuterung auf die vorstehende Beschreibung verwiesen wird. Im Falle der vorstehenden Überdrucksituation, bei der die Überdruckventilanordnung 11 vollständig öffnet, siehe Figur 8 D, erzeugt die durch den Strömungskanal 14 hindurchtretende, Druckausgleichs-getriebene Luftströmung bei Passieren der Pfeife 22 einen unüberhörbaren Signalton.

Das dynamische Auslenkverhalten der Ventilklappe, sowohl im Falle der Ventilöffnung als auch im Falle der Rückführung der geöffneten Ventilklappe in die geschlossene Stellung, ist beeinflussbar durch die Vorkrümmung der Ventilklappe sowie durch die Material- und Formeigenschaften der flächigen, elastischen Ventilklappe, wie Dicke, Steifigkeit und Dehnbarkeit der vorzugsweise aus Elastomer-Material gefertigten Ventilklappe. Benutzt man steifere oder weichere bzw. dickere oder dünnere Ventilklappen, öffnet sich das Ventil bei einem niedrigeren oder höheren Druck. Je nach Patienten-Typ (Mann/Frau/Kind) und/oder physiologischer Patientenkonstitution ist eine geeignete Ventilklappe in die hierfür vorgesehene schlitzartige Einschuböffnung der Überdruckventilanordnung einzusetzen. Der behandelnde Arzt kann hierzu auf eine Reihe unterschiedlich konfektionierter Ventilklappen zurückgreifen, die als austauschbare Einsatzmodule ausgebildet und bevorratet sind.

### Bezugszeichenliste

- 1: Durchströmungskörper
- 2: Durchströmungskörperende
- 3: Inspiratorische Luftzuströmung
- 3': Respiratorische Luftabströmung
- 4: Unteres Durchströmungskörperende
- 5: Verbindungsstruktur
- 6: Tracheostomiekanüle
- 7: Gitterstruktur
- 8: Durchströmungskörperwand
- 9: Durchströmungskanal
- 10: Öffnung
- 11: Überdruckventilanordnung
- 12: Ventilöffnung
- 13: Strömungskanalwand
- 13': Öffnung des Strömungskanals
- 14: Strömungskanal
- 15: Ventilklappe
- 16: Befestigungspin
- 17: Ventilklappenrand
- 18: Unterer Ventilöffnungsrand
- 19: Schwellenflanke
- 20: Nutförmige Ausnehmung
- 21: Signalerzeugungsmittel
- 22: Pfeife
- 23: Einwegklappenventilanordnung
- 24: Längserstreckung des Durchströmungskanals
- 25: Schlitzförmige Ausnehmung
- 26: Befestigungselementes
- 27: Kulissenwänden
- 28,29: Schwellenstrukturen
- L: Luftströmung
- Ü: Überdruck
- U: Unterdruck, Einatmung

## Patentansprüche

1. Sprechventil-Anordnung zum Aufsetzen und Anbringen an eine Tracheostomiekanüle (6), mit einem einen Durchströmungskanal (9) umschliessenden Durchströmungskörper (1), der eine Durchströmungskörperwand (8) aufweist, die den Durchströmungskanal (9) radial einschließt, und der einseitig endseitig eine geeignet ausgebildete Verbindungsstruktur (5) zur Fügung an ein proximales Ende der Tracheostomiekanüle (6) aufweist, an dessen der Verbindungsstruktur (5) längs des Durchströmungskanals (9) gegenüberliegenden Ende ein Einwegventil (23) angeordnet ist und längs des Durchströmungskanals (9) jeweils räumlich beabstandet zwischen der Verbindungsstruktur (5) und dem Einwegventil (23) eine durch eine Überdruckventilanordnung (11) abschließbare Ventilöffnung (10, 12) angeordnet ist,
wobei die Ventilöffnung (10) die Durchströmungskörperwand (8) vollständig durchsetzt, und
dass die Überdruckventilanordnung (11) wenigstens eine elastisch verformbare Ventilklappe (15) aufweist, die in einem ersten Zustand die zwischen der Verbindungsstruktur (5) und dem Einwegventil (23) am Durchströmungskörper (1) vorgesehene Ventilöffnung (10, 12) abzudecken vermag und bei einem vorgebbaren Überdruck innerhalb des Durchströmungskanals (9) durch eine Überdruck getriebene, elastische Verformung der Ventilklappe (15) in einen die Ventilöffnung (10,12) zumindest teilweise freigebenden zweiten Zustand überführbar ist.

2. Anordnung nach dem Anspruch 1,
wobei mittel- oder unmittelbar an der Überdruckventilanordnung (11) oder stromab zur Überdruckventilanordnung (11) ein Signalerzeugungsmittel (21) angeordnet ist, das im Falle eines Überdruck-getriebenen Öffnens der Überdruckventilanordnung (11) ein Signal erzeugt.

3. Anordnung nach Anspruch 2,
wobei das Signalerzeugungsmittel (21) zur Erzeugung eines akustisch wahrnehmbaren Schallsignals ausgebildet ist.

4. Anordnung nach Anspruch 3,
wobei das Signalerzeugungsmittel (21) das Schallsignal durch Schwingungsanregung einer die Überdruckventilanordnung passierenden Luftströmung erzeugt.

5. Anordnung nach Anspruch 3 oder 4,
wobei sich stromab zu der durch die Überdruckventilanordnung (11) abschließbaren Ventilöffnung (10, 12) ein Strömungskanal (14) anschließt, längs dem das Signalerzeugungsmittel (21) angeordnet ist.

6. Anordnung nach einem der Ansprüche 3 bis 5,
wobeiss das Signalerzeugungsmittel (21) in Art einer Pfeife (22) ausgebildet ist.

7. Anordnung nach Anspruch 6,
wobei die Pfeife (22) in Art einer Labial- oder Lingualpfeife ausgebildet ist.

8. Anordnung nach einem der Ansprüche 2 oder 3,
wobei das Signalerzeugungsmittel (21) ein elektrisches, elektromagnetisches oder optisches Signal zu erzeugen in der Lage ist, und
dass das Signalerzeugungsmittel (21) mit einer Überwachungseinheit kabel- oder kabellos verbunden ist, an die das Signal übertragbar ist.

9. Anordnung nach einem der Ansprüche 5 bis 8,
wobei der Strömungskanal (14) von einer Strömungskanalwand (13) oder vom Durchströmungskörper (1) und von einer Strömungskanalwand (13) begrenzt ist.

10. Anordnung nach einem der Ansprüche 1 bis Anspruch 9,
wobei die wenigstens eine elastisch verformbare Ventilklappe (15) über eine Form und/oder Eigenelastizität verfügt, die eine Rückstellkraft basierte Wirkung entfalten, durch die die elastisch verformbare Ventilklappe (15) aus dem zweiten Zustand in den ersten Zustand zumindest teilweise überführbar ist.

11. Anordnung nach einem der Ansprüche 1 bis 10,
wobei der Ventilöffnung (10, 12) zwei Halbräume zuordenbar sind,
dass die elastisch verformbare Ventilklappe (15) innerhalb eines der beiden Halbräume wenigstens abschnittsweise ortsfest relativ zur Ventilöffnung (10, 12) gefügt ist, und dass die elastisch verformbare Ventilklappe (15) in dem anderen der beiden Halbräume lose zur Ventilöffnung (10, 12) gelagert ist.

12. Anordnung nach einem der Ansprüche 1 bis 11,
wobei die wenigstens eine elastisch verformbare Ventilklappe (15) am Durchströmungskörper (1) und/oder an der Überdruckventilanordnung (11) außerhalb des Durchströmungskörpers (1) austauschbar gefügt ist.

13. Anordnung nach einem der Ansprüche 1 bis 12,
wobei wenigstens zwei elastisch verformbare Ventilklappen (15) bevorratet sind, die sich wenigstens in Form, Größe und/oder in ihren elastischen Eigenschaften voneinander unterscheiden, von denen wenigstens eine in Abhängigkeit Beatmungsphysiologischer Patienteneigenschaften am Durchströmungskörper (1) und/oder an der Überdruckventilanordnung (11) fügbar ist.

14. Anordnung nach einem der Ansprüche 11 bis 13,
wobei innerhalb des zweiten Halbraumes die wenigstens eine elastisch verformbare Ventilklappe (15) im ersten Zustand zumindest abschnittsweise mit einem randseitigen Übermaß die Ventilöffnung (10, 12) abdeckt, d.h. die Ventilklappe (15) erstreckt sich zumindest abschnittsweise über die Ventilöffnungsweite hinaus.

15. Anordnung nach Anspruch 14,
wobei das randseitige Übermaß an wenigstens einer Stelle minimal ist.

16. Anordnung nach einem der Ansprüche 1 bis 15,
wobei die wenigstens eine elastisch verformbare Ventilklappe (15) flächig ausgebildet ist und im ersten Zustand eine Flächennormale besitzt, die mit einer dem Durchströmungskanal (9) zuordenbaren Durchströmungskanallängsachse wenigstens einen Winkel α einschließt, für den gilt: 80° ≤ α ≤ 100°.

17. Anordnung nach einem der Ansprüche 1 bis 16,
wobei die flächige Ventilklappe (15) im ersten Zustand eine zum Durchströmungskanal (9) zugewandt orientierte konvexe Flächenkrümmung aufweist.

## Claims

1. Speaking valve arrangement for fitting and attachment to a tracheostomy cannula (6), with a through-flow body (1) enclosing a through-flow channel (9), which has a through-flow body wall (8) that radially encloses the through-flow channel (9), and which has a suitably designed connecting structure (5) at one end for joining to a proximal end of the tracheostomy cannula (6), at the end of which structure, located opposite to the connecting structure (5) along the through-flow channel (9), is arranged a one-way valve (23), and a valve opening (10, 12) that can be closed by a pressure-relief valve arrangement (11) is arranged along the through-flow channel (9), spatially separated between the connecting structure (5) and the one-way valve (23), wherein
the valve opening (10) passes completely through the through-flow body wall (8), and the pressure-relief valve arrangement (11) has at least one elastically deformable valve flap (15), which in a first state is able to cover the valve opening (10, 12) provided between the connecting structure (5) and the one-way valve (23) on the through-flow body (1), and in the event of a predefinable overpressure within the flow channel (9), can be converted into a second state, at least partially releasing the valve opening (10, 12) by an overpressure-driven elastic deformation of the valve flap (15).

2. Arrangement in accordance with Claim 1, wherein
a signal generating means (21) is arranged directly or indirectly on the pressure-relief valve arrangement (11), or downstream of the pressure-relief valve arrangement (11), which in the event of an overpressure-driven opening of the pressure-relief valve arrangement (11) generates a signal.

3. Arrangement in accordance with Claim 2, wherein
the signal generating means (21) is designed to generate an acoustically perceptible sound signal.

4. Arrangement in accordance with Claim 3, wherein
the signal generating means (21) generates the sound signal by the vibrational excitation of an air flow passing through the pressure-relief valve arrangement.

5. Arrangement in accordance with Claim 3 or 4, wherein
downstream of the valve opening (10, 12) that can be closed by the pressure-relief valve arrangement (11) is connected a flow channel (14), along which is arranged the signal generating means (21).

6. Arrangement in accordance with one of the Claims 3 to 5, wherein
the signal generating means (21) is designed in the form of a whistle (22).

7. Arrangement in accordance with Claim 6, wherein
the whistle (22) is designed in the form of a labial or lingual whistle.

8. Arrangement in accordance with one of the Claims 2 or 3, wherein
the signal generating means (21) is capable of generating an electrical, electromagnetic or optical signal, and the signal generating means (21) is connected by cable or wirelessly to a monitoring unit, to which the signal can be transmitted.

9. Arrangement in accordance with one of the Claims 5 to 8, wherein
the flow channel (14) is bounded by a flow channel wall (13), or by the through-flow body (1) and by a flow channel wall (13).

10. Arrangement in accordance with one of the Claims 1 to 9, wherein
the at least one elastically deformable valve flap (15) has a shape and/or an inherent elasticity that develops a restoring force-based effect, by means of which the elastically deformable valve flap (15) can be at least partially translated from the second state into the first state.

11. Arrangement in accordance with one of the Claims 1 to 10, wherein
two half-chambers can be assigned to the valve opening (10, 12), and the elastically deformable valve flap (15) is joined within one of the two half-chambers, at least in sections, in a fixed position relative to the valve opening (10, 12), and the elastically deformable valve flap (15) is mounted loosely relative to the valve opening (10, 12) in the other of the two half-chambers.

12. Arrangement in accordance with one of the Claims 1 to 11, wherein
. the at least one elastically deformable valve flap (15) is interchangeably joined to the through-flow body (1) and/or to the pressure-relief valve arrangement (11) outside the through-flow body (1).

13. Arrangement in accordance with one of the Claims 1 to 12, wherein
. at least two elastically deformable valve flaps (15) are provided, which differ from one another at least in shape, size. and/or in their elastic properties, of which at least one can be joined to the through-flow body (1) and/or to the pressure-relief valve arrangement (11). depending on the physiological properties of the patient in terms of breathing.

14. Arrangement in accordance with one of the Claims 11 to 13, wherein
within the second half-chamber, the at least one elastically deformable valve flap (15) covers the valve opening (10, 12) in the first state, at least in sections, with an oversized edge, that is to say, the valve flap (15) extends, at least in sections, beyond the latter.

15. Arrangement in accordance with Claim 14, wherein
the oversize of the edge is a minimum at at least one location.

16. Arrangement in accordance with one of the Claims 1 to 15, wherein
the at least one elastically deformable valve flap (15) has a planar design, and in the first state has a surface normal, which forms at least an angle α with a longitudinal through-flow channel axis that can be assigned to the through-flow channel (9), for which the following applies: 80° ≤ α ≤ 100°.

17. Arrangement in accordance with one of the Claims 1 to 16, wherein
in the first state the planar valve flap (15) has a convex surface curvature orientated towards the through-flow channel (9).

## Revendications

1. Dispositif de valve de phonation, destiné à être posé et monté sur une canule de trachéotomie (6), pourvu d'un organe de passage (1), entourant une canalisation de passage (9), qui comporte une paroi (8) d'organe de passage, qui inclut en direction radiale la canalisation de passage (9) et qui d'un côté comporte sur le côté extrémité une structure d'assemblage (5), conçue de manière adaptée, destinée à être reliée sur une extrémité proximale de la canule de trachéotomie (6), sur l'extrémité opposée à la structure d'assemblage (5) de laquelle, le long de la canalisation de passage (9) est placée une valve unidirectionnelle (23) et le long de la canalisation de passage (9), chaque fois avec un écart physique entre la structure d'assemblage (5) et la valve unidirectionnelle (23) est placée une ouverture de valve (10, 12) pouvant se fermer par un dispositif (11) de valve de surpression,
l'ouverture de valve (10) traversant totalement la paroi (8) d'organe de passage et
le dispositif (11) de valve de surpression comportant au moins un clapet de valve (15) élastiquement déformable, qui dans une première position, est susceptible de recouvrir l'ouverture de valve (10, 12) prévue sur l'organe de passage (1), entre la structure d'assemblage (5) et la valve unidirectionnelle (23) et lors d'une surpression définissable à l'intérieur de la canalisation de passage (9), est susceptible d'être passé dans une deuxième position, libérant au moins partiellement l'ouverture de valve (10, 12), par une déformation élastique du clapet de valve (15), générée par une surpression.

2. Dispositif selon la revendication 1,
indirectement ou directement sur le dispositif (11) de valve de surpression ou en aval du dispositif (11) de valve de surpression étant placé un moyen générateur de signaux (21), qui dans le cas d'une ouverture du dispositif (11) de valve de surpression générée par une surpression, génère un signal.

3. Dispositif selon la revendication 2,
le moyen générateur de signaux (21) étant conçu pour générer un signal sonore, acoustiquement perceptible.

4. Dispositif selon la revendication 3,
le moyen générateur de signaux (21) générant le signal sonore par excitation vibratoire d'un courant d'air passant dans le dispositif de valve de surpression.

5. Dispositif selon la revendication 3 ou 4,
en aval de l'ouverture de valve (10, 12) susceptible d'être fermée par le dispositif (11) de valve de surpression se raccordant une canalisation d'écoulement (14), le long de laquelle est placé le moyen générateur de signaux (21).

6. Dispositif selon l'une quelconque des revendications 3 à 5,
le moyen générateur de signaux (21) étant conçu à la manière d'un sifflet (22).

7. Dispositif selon la revendication 6,
la sifflet (22) étant conçu à la manière d'une sifflet labial ou d'un sifflet lingual.

8. Dispositif selon l'une quelconque des revendications 2 ou 3,
le moyen générateur de signaux (21) étant apte à générer un signal électrique, électromagnétique ou optique et
le moyen générateur de signaux (21) étant connecté par câble ou sans câble avec une unité de supervision, à laquelle le signal est transmissible.

9. Dispositif selon l'une quelconque des revendications 5 à 8,
la canalisation d'écoulement (14) étant délimitée par une paroi (13) de canalisation d'écoulement ou par l'organe de passage (1) et par une paroi (13) de canalisation d'écoulement.

10. Dispositif selon l'une quelconque des revendications 1 à la revendication 9,
l'au moins un clapet de valve (15) élastiquement déformable disposant d'une élasticité de forme et / ou d'une élasticité propre, qui déploient un effet basé sur une force de rappel, par lequel le clapet de valve (15) élastiquement déformable peut être passé au moins partiellement de la deuxième position dans la première position.

11. Dispositif selon l'une quelconque des revendications 1 à 10,
à l'ouverture de valve (10, 12) pouvant être associés deux demi-espaces,
le clapet de valve (15) élastiquement déformable étant relié à l'intérieur de l'un des deux demi-espaces au moins par endroits de manière stationnaire par rapport à l'ouverture de valve (10, 12) et le clapet de valve (15) élastiquement déformable étant logé dans l'autre des deux demi-espaces de manière mobile, par rapport à l'ouverture de valve (10, 12).

12. Dispositif selon l'une quelconque des revendications 1 à 11,
l'au moins un clapet de valve (15) élastiquement déformable étant relié de manière interchangeable sur l'organe de passage (1) et / ou sur le dispositif (11) de valve de surpression, à l'extérieur de l'organe de passage (1).

13. Dispositif selon l'une quelconque des revendications 1 à 12,
au moins deux clapets de valve (15) élastiquement déformables étant tenus en réserve, qui se distinguent les uns des autres au moins au niveau de leur forme, de leur taille et / ou de leurs propriétés élastiques, dont au moins l'un est susceptible d'être relié sur l'organe de passage (1) et / ou sur le dispositif (11) de valve de surpression en fonction des propriétés physiologiques de ventilation du patient.

14. Dispositif selon l'une quelconque des revendications 11 à 13,
à l'intérieur du deuxième demi-espace, l'au moins un clapet de valve (15) élastiquement déformable recouvrant dans la première position au moins par endroit l'ouverture de valve (10, 12) par un surdimensionnement sur son bord, c'est-à-dire que le clapet de valve (15) s'étend au moins par endroits au-delà de l'ouverture de valve.

15. Dispositif selon la revendication 14,
le surdimensionnement sur le bord étant minimal sur au moins un endroit.

16. Dispositif selon l'une quelconque des revendications 1 à 15,
l'au moins un clapet de valve (15) élastiquement déformable étant conçu de forme plane et dans la première position, possédant une normale à la surface, qui avec un axe longitudinal de passage susceptible d'être associé à la canalisation de passage (9), inclut au moins un angle α pour lequel s'applique : 80° ≤ α ≤ 100 °.

17. Dispositif selon l'une quelconque des revendications 1 à 16,
le clapet de valve (15) plan comportant dans la première position un courbure de surface convexe, orientée de sorte à faire face à la canalisation de passage (9).
